(19) Europäisches Patentamt
European Patent Office
Office européen des brevets

(11) Veröffentlichungsnummer: **0 139 993**
**A2**

# (12) EUROPÄISCHE PATENTANMELDUNG

(21) Anmeldenummer: **84110120.7**

(22) Anmeldetag: **24.08.84**

(51) Int. Cl.[4]: **C 07 D 239/95**
C 07 D 417/12, C 07 D 403/12
A 61 K 31/495, A 61 K 31/41
A 61 K 31/425

(30) Priorität: **06.10.83 DE 3336409**

(43) Veröffentlichungstag der Anmeldung:
**08.05.85 Patentblatt 85/19**

(84) Benannte Vertragsstaaten:
**AT BE CH DE FR GB IT LI LU NL SE**

(71) Anmelder: **LUDWIG HEUMANN & CO GMBH**
**Hagelloffstrasse 18-28**
**D-8500 Nürnberg(DE)**

(72) Erfinder: **Schickander, Helmut, Dr. Dipl.-Chem.**
**Moosäcker 25**
**D-8501 Eckental(DE)**

(72) Erfinder: **Szelenyi, Istan, Dr.**
**Brahmsstrasse 16**
**D-8501 Schwaig(DE)**

(72) Erfinder: **Mörsdorf, Peter, Dr. Dipl.-Chem.**
**Untere Bahnhofstrasse 16**
**D-8501 Cadolzburg(DE)**

(72) Erfinder: **Ahrens, Kurt Henning, Dr.**
**Praterstrasse 9**
**D-8500 Nürnberg(DE)**

(74) Vertreter: **Kraus, Walter, Dr. et al,**
**Patentanwälte Kraus, Weisert & Partner Irmgardstrasse 15**
**D-8000 München 71(DE)**

(54) **Chinazolinderivate, Verfahren zu ihrer Herstellung und diese Verbindungen enthaltende Arzneimittel.**

(57) Es werden Chinazolinderivate der allgemeinen Formel I beschrieben

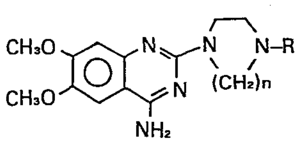

in der R für einen ein- bis dreifach substituierten Fünfringheterocyclus aus der Gruppe Triazole, Oxadiazole, Thiazole und Imidazole steht, wobei der Heterocyclus mit dem Piperazin- bzw. Homopiperazinring über ein Kohlenstoffatom verbunden ist und n den Wert 2 oder 3 hat, oder R eine N-Cyanophenylimidocarbonatgruppe bedeutet, sowie die physiologisch annehmbaren Salze davon, Verfahren zur ihrer Herstellung und diese Verbindungen enthaltende Arzneimittel.

EP 0 139 993 A2

Croydon Printing Company Ltd.

BESCHREIBUNG

Die Erfindung betrifft neue Chinazolinderivate mit antihypertensiver Wirkung, ein Verfahren zu ihrer Herstellung und Arzneimittel, die diese Verbindungen enthalten, sowie schließlich die Verwendung dieser Verbindungen in der Therapie.

Ein Wirkstoff aus der Klasse der Chinazolinderivate, "PRAZOSIN", wird bereits, da er eine selektive Blockade der $\alpha_1$-Rezeptoren bewirkt, ohne $\alpha_2$-Rezeptoren zu blockieren, in der Therapie als Antihypertensivum verwendet.

Der Erfindung liegt die Aufgabe zugrunde, neue Verbindungen mit verbesserter antihypertensiver Wirksamkeit zur Verfügung zu stellen.

Diese Aufgabe wird durch die Erfindung gelöst.

Gegenstand der Erfindung sind daher neue Chinazolinderivate der allgemeinen Formel I

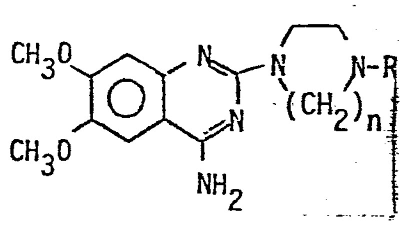

(I)

in der

R für einen ein- bis dreifach substituierten Fünfringheterocyclus aus der Gruppe Triazole, Oxadiazole, Thiazole und Imidazole steht, wobei der Heterocyclus mit dem Piperazin- bzw. Homopiperazinring über ein Kohlenstoffatom verbunden ist und n den Wert 2 oder 3 hat, oder R eine N-Cyanophenylimidocarbonatgruppe bedeutet, sowie die physiologisch annehmbaren Salze davon.

In der allgemeinen Formel I bedeutet R einen ein- bis dreifach substituierten Fünfringheterocyclus, nämlich ein Triazol, Oxadiazol, Thiazol oder Imidazol. Beispiele hierfür sind 1,2,4-Triazol, 1,2,4-Oxadiazol, 1,3-Thiazol und 1,3-Imidazol.

Der genannte Fünfringheterocyclus kann ein- bis dreifach substituiert sein, wobei im Falle des Triazolrings und des Thiazolrings die Zweifachsubstitution bevorzugt wird. Im Falle des Oxadiazolrings und des Imidazolrings wird die Einfachsubstitution bevorzugt.

Als Substituenten hierfür kommen zum Beispiel folgende Gruppen in Betracht: Niedrigalkylgruppen, Niedrigalkoxygruppen, Carboxyalkylgruppen und gegebenenfalls substituierte, insbesondere niedrigalkylsubstituierte, Aminogruppen. Bevorzugt werden Niedrigalkylgruppen und unsubstituierte Aminogruppen.

Hierin werden unter "Niedrigalkylgruppen", "Niedrigalkoxygruppen" etc. Gruppen mit 1 bis 4 Kohlenstoffatomen im Alkylteil verstanden. Beispiele hierfür sind Methylgruppen, Ethylgruppen und Isopropylgruppen, wobei Methyl- und Ethylgruppen bevorzugt werden.

So bedeutet beispielsweise in der allgemeinen Formel I R einen 1,2,4-Triazolring, der in Position 3 durch eine Aminogruppe substituiert ist, die gegebenenfalls lineare oder verzweigtkettige Niedrigalkylreste tragen kann. Eine solche Gruppe ist zum Beispiel die Dimethylaminogruppe.

Weiterhin kann beispielsweise R ein 1,2,4-Triazolring sein, der in Position 1 durch Niedrigalkylgruppen, vorzugsweise Methyl- oder Ethylgruppen, und in Position 3 oder 5 durch eine Aminogruppe, die gegebenenfalls lineare oder verzweigtkettige Niedrigalkylreste tragen kann, substituiert ist. Ein

1,2,4-Triazolring mit einer Zweifachsubstitution in den Positionen 1 und 3 des aromatischen heterocyclischen Systems wird dabei bevorzugt.

Weiterhin kann beispielsweise R auch ein 1,2,5-Thiadiazol-1-oxidring sein, der in Position 4 durch eine Niedrigalkoxygruppe, bevorzugt eine Methoxygruppe oder Ethoxygruppe, substituiert ist.

Im Falle, daß R für einen 1,3-Thiazolring steht, kann dieser Heterocyclus in Position 4 durch eine Aminogruppe und in Position 5 durch eine Carboxyalkylgruppe, vorzugsweise Carboxyethylgruppe, substituiert sein.

Schließlich kann R auch die Gruppe 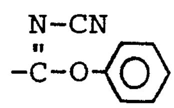 bedeuten.

Die Erfindung umfaßt auch die physiologisch annehmbaren Salze dieser neuen Chinazolinderivate.

Diese Salze können zum Beispiel mit Mineralsäuren, wie Chlor-, Brom- und Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure etc., gebildet werden.

Die Erfindung umschließt weiterhin alle tautomeren Formen und deren Salze. Die erfindungsgemäßen Verbindungen können Di- und Trisalze sowie Hydrate bilden, die gleichfalls unter den Rahmen der vorliegenden Erfindung fallen.

Die erfindungsgemäßen Verbindungen werden durch ein Verfahren hergestellt, das dadurch gekennzeichnet ist, daß man

a) zur Herstellung von Verbindungen der allgemeinen Formel I, in der R für die Gruppe 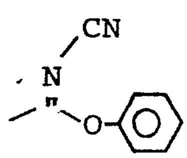

steht, eine Verbindung der allgemeinen Formel II

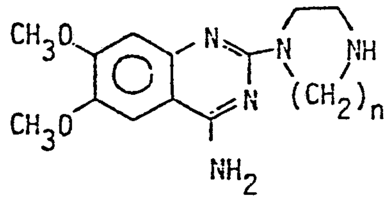

(II)

in der n wie oben definiert ist, mit äquimolaren Mengen N-Cyanodiphenylimidocarbonat der Formel III

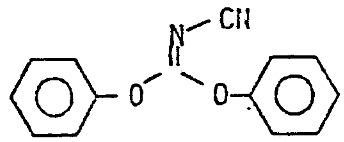

(III)

in einem Alkohol, vorzugsweise in Isopropanol, bei 25 bis 82°C, vorzugsweise bei Raumtemperatur, innerhalb von 80 Minuten zu der erfindungsgemäßen Verbindung der allgemeinen Formel Ia

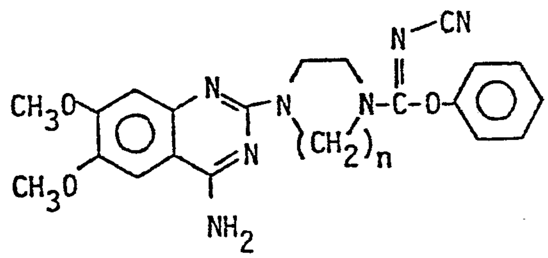

(Ia)

in der n wie oben definiert ist,

umsetzt oder daß man

b) zur Herstellung von Verbindungen der allgemeinen Formel I, in der R für ein 1,2,4-Triazol oder 1,3-Thiazol steht, eine Verbindung der allgemeinen Formel Ia in einem alkoholischen Lösungsmittel, vorzugsweise Methanol, bei Rückflußtemperatur mit einem Hydrazinderivat oder Mercaptoessigsäureester zu einer erfindungsgemäßen Verbindung der allgemeinen Formel Ic, Id, Ie oder If

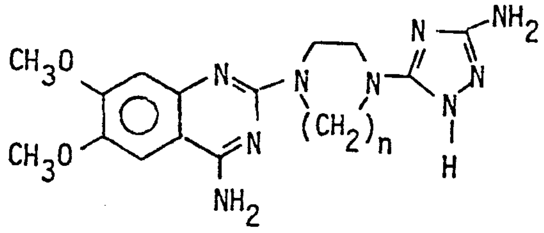

(Ic)

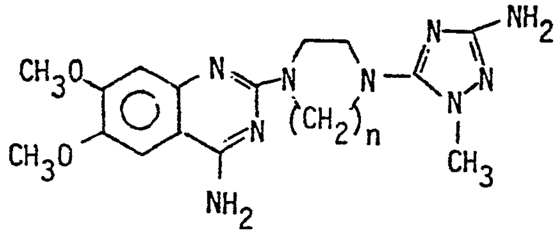

(Id)

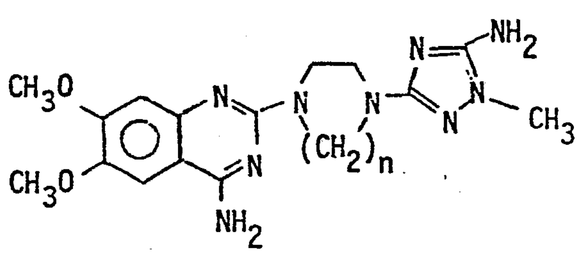

(Ie)

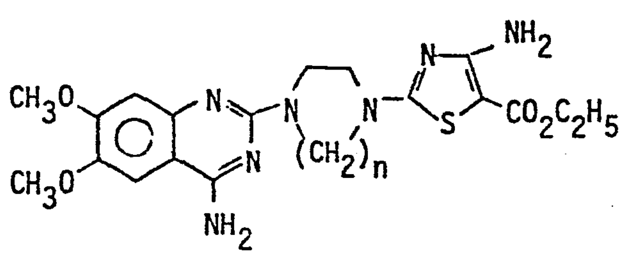

(If)

umsetzt und daß man

gegebenenfalls die in Stufe a) oder b) erhaltene Verbindung in ihr physiologisch annehmbares Salz umwandelt.

Die Isolierung der erhaltenen Verbindung der allgemeinen Formel I erfolgt in üblicher Weise, beispielsweise durch Kristallisation.

Die erhaltene Verbindung der allgemeinen Formel I kann in an sich bekannter Weise mit einer pharmakologisch annehmbaren Säure in ihr Salz umgewandelt werden.

Die erfindungsgemäßen Verbindungen, vorzugsweise in der Form eines Salzes, können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmakologisch annehmbaren Trägern oder Verdünnungsmitteln hergestellt werden.

Die erfindungsgemäßen Verbindungen können daher für die orale, bukkale und parenterale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/ oder Dispergierungsmittel, enthalten. Alternativ kann der Wirkstoff auch

in Pulverform zur Rekonstitution mit einem geeigneten Träger, zum Beispiel sterilem, pyrogenfreiem Wasser, vor dem Gebrauch vorliegen.

Für die orale Verabreichung ist eine geeignete Tagesdosis an erfindungsgemäßen Verbindungen 1 bis 4 Dosen bis insgesamt 1/3 mg bis 20 mg/Tag je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die Dosierung bei intravenöser Gabe liegt bei etwa 1/5 bis 1/10 der oralen Tagesdosis.

Pharmakologische Aktivität

Die erfindungsgemäßen neuen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen.

Eine anerkannte Methode ist die Bestimmung der blutdrucksenkenden Wirkung an spontanhypertensiven Ratten. Es wurden dabei Ratten mit einem Ausgangsgewicht von 160 - 185 g verwendet. Die Blutdruckmessung erfolgte unblutig mit Hilfe eines BP Recorders (Typ 8005, w + w electronic AG., Basel).

An gesunden Ratten (SIV 50, Fa. Dr. Ivanovas, Kisslegg, 160 - 180 g) wurde ein mittlerer systolischer Blutdruckwert von 125 $\pm$ 6 mm Hg (n = 10) ermittelt. Die blutdrucksenkende Wirkung der genannten Verbindungen wurde unter Berücksichtigung des normalen systolischen Wertes in % angegeben, wobei eine 100 %-ige Wirkung die Senkung des

Blutdrucks auf den von uns gefundenen normalen Wert von 125 mm Hg bedeutet.
Als Vergleichssubstanz diente Prazosin.

$ED_{50}$ (Prazosin)   0,7 mg /kg p.o.

$ED_{50}$ (Beispiel 4)   0,17 mg /kg p.o.

Die anderen Beispiele zeigen ähnliche pharmakologische Aktivitäten.

Die Erfindung wird anhand der folgenden Beispiele erläutert.

## Beispiel 1

N-Cyano-[(4-amino-6,7-dimethoxychinazolin-2-yl)-N,N-tetramethylen-4-imino-]-phenylisoharnstoff

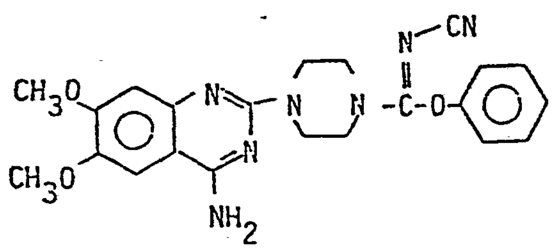

2.89 g (10 mmol) 4-Amino-6,7-dimethoxy-2-piperazinochinazolin und 2.38 g (10 mmol) N-Cyano-diphenylimidocarbonat werden in 100 ml Isopropanol suspendiert und 3 Stunden auf 50 °C erwärmt. Anschließend läßt man das Reaktionsgemisch abkühlen und trennt den Feststoff ab.

Farblose Kristalle vom Schmelzpunkt: 243 - 244 °C

Ausbeute: 3,77 g (87 %)

Rf: 0.64 (Essigester/Ethanol 7 : 3)

$C_{22}H_{23}N_7O_3$ (433)

$^1$H-NMR-Daten: (d$_6$-DMSO, TMS als interner Standard)

$\delta$ = 3.50 - 4.13 (m) (2x$OC\underline{H}_3$; 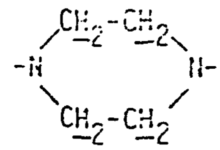 ) 14

6.77 (s) (Aromaten-$\underline{H}$) 1 H,

7.03 - 7.63 (m) (Aromaten-$\underline{H}$, -$N\underline{H}_2$) 8 H ppm.

Beispiel 2

3-Ethoxy-4-[4-(4-amino-6,7-dimethoxychinazolin-2-yl)-piperazin-1-yl]-1,2,5-thiadiazol-1-oxid

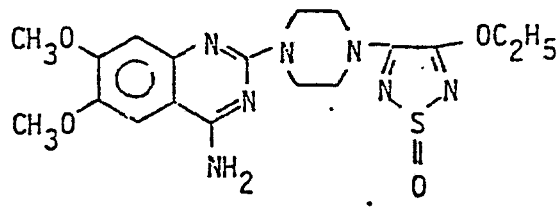

Zu 2.9 g (10 mmol) 4-Amino-6,7-dimethoxy-2-piperazinochinazolin, gelöst in 50 ml Tetrahydrofuran und 10 ml Methanol, werden 1.9 g (10 mmol) 3,4-Diethoxy-1,2,5-thiadiazol-1-oxid gegeben und 5 Stunden bei Raumtemperatur gerührt. Der anfallende Feststoff wird abgetrennt und aus 15 ml Dimethylformamid und 10 ml Ethanol umkristallisiert.

Gelbe Kristalle vom Schmelzpunkt 234 - 235 °C

Ausbeute: 3.01 g (70 % d.Th.)

Rf: 0.57 (Essigester / Ethanol 70:30 )

$C_{18}H_{23}N_7O_4S$ (433)

$^1$H-NMR-Spektrum: (d$_6$-DMSO, TMS als interner Standard)

$\delta$ = 1.43 (t) ($CH_3$-$CH_2$) 3 H,
3.60 - 4.10 (m) (2 x N ($CH_2$)$_2$; (2 x $OCH_3$) 14 H,
4.5 (q) ($CH_3CH_2$) 2 H,
6.77 (s) (Aromaten-H) 1 H,
7.13 (s) (-$NH_2$) 2 H (austauschbar mit $D_2O$)
7.47 (s) (Aromaten-H) 1 H ppm.

## Beispiel 3

4-Amino-2-[4-(3-amino-1H-1,2,4-triazol-5-yl)-piperazin-1-yl]-6,7-dimethoxychinazolin

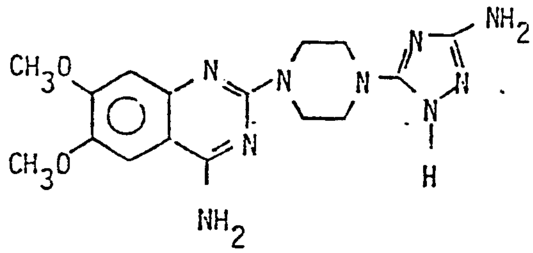

4.33 g (10 mmol) N-Cyano-[(4-amino-6,7-dimethoxychinazolin-2-yl)-N,N-tetramethylen-4-imino]-phenylisoharnstoff werden mit 0,5 g (10 mmol) Hydrazinhydrat in 20 ml Methanol gelöst und 20 Minuten auf Rückflußtemperatur erhitzt, anschließend wird auf Raumtemperatur abgekühlt und die Reaktionslösung im Vakuum eingeengt. Der Rückstand wird aus Methanol umkristallisiert.

Farblose Kristalle vom Schmelzpunkt 172 - 173 °C (zers.)

Ausbeute: 2.94 g (79 % d.Th.)

Rf: 0.65 (Methanol/$NH_3$-Konz. 99 : 1)

$C_{16}H_{21}N_9O_2$ (371)

$^1$H-NMR-Spektrum: (d$_6$-DMSO, TMS als interner Standard)

$\delta$ = 3.27 (m) (-N $(CH_2)_2$) 4 H,

3.63 - 4.00 (m) (N$(-CH_2)_2$) 4 H,

3.80 (s) (-$OCH_3$) 3 H,

3.85 (s) (-$OCH_3$) 3 H,

5.63 (s, breit) ($NH_2$) 2 H (austauschbar mit $D_2O$)

6.78 (s) (Aromaten-H) 1 H,

7.12 (s, breit) ($NH_2$) 2 H (austauschbar mit $D_2O$),

7.47 (s) (Aromaten-H) 1 H,

11.57 (s, breit) (N-H) (austauschbar mit $D_2O$ ppm.

Beispiel 4

4-Amino-2-[4-(5-amino-1-methyl-1H-1,2,4-triazol-5-yl)-piperazin-1-yl]-6,7-dimethoxychinazolin

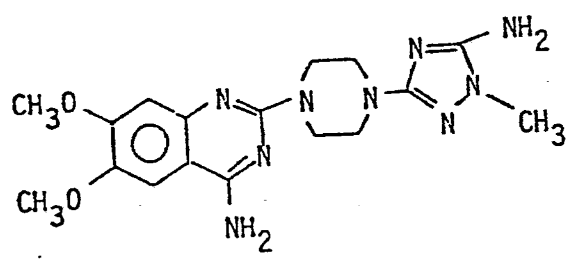

Die Herstellung erfolgt analog Beispiel 3 aus 4.33 g (10 mmol) N-Cyano-[(4-amino-6,7-dimethoxychinazolin-2-yl)-N,N-tetramethylen-4-imino]-phenylisoharnstoff und 0.46 g (10 mmol) Methylhydrazin.

Farblose Kristalle vom Schmelzpunkt > 280 °C

Rf: 0.32 (EtOAc/MeOH 50:50 )

| | | | | |
|---|---|---|---|---|
| $C_{17}H_{23}O_2N_9$ (385) · 1/2 $H_2O$ | Ber.: | C 51,78 | H 6,59 | N 31,98 |
| | Gef.: | C 51,80 | H 6,18 | N 31,54 |

$^1$H-NMR-Spektrum: (d$_6$-DMSO, TMS als interner Standard)

$\delta$ = 3.23 (m) ($N<^{C\underline{H}_2}_{C\underline{H}_2}$) 4 H,

3.33 (s) (N-C$\underline{H}_3$) 3 H,

3.57 - 4.03 (m) ($N<^{C\underline{H}_2}_{C\underline{H}_2}$) 4 H,

3.80 (s) (OC$\underline{H}_3$) 3 H,

3.83 (s) (OC$\underline{H}_3$) 3 H,

6.00 (s) (-N$\underline{H}_2$) 2 H (austauschbar mit $D_2O$),

6.77 (s) (Aromaten-$\underline{H}$) 1 H,

7.10 (s, breit) (-N$\underline{H}_2$) 2 H (austauschbar mit $D_2O$)

7.47 (s) (Aromaten-$\underline{H}$) 1 H, ppm.

## Beispiel 5

4-Amino-2-[4-(3-amino-1-methyl-1H-1,2,4-triazol-5-yl)-piperazin-1-yl]-6,7-dimethoxychinazolin

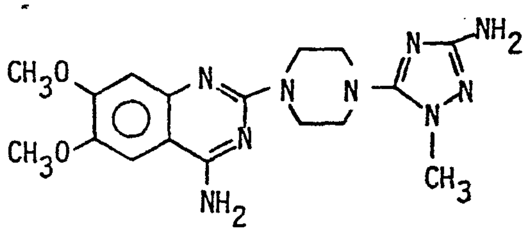

Die Herstellung erfolgt analog Beispiel 3 aus 4.33 g (10 mmol) N-Cyano-[(4-amino-6,7-dimethoxychinazolin-2-yl)-N,N-tetramethylen-4-imino]-phenylisoharnstoff und 0.46 g (10 mmol) Methylhydrazin. Nach dem Einengen der Reaktionslösung wird die Verbindung durch fraktionierte Kristallisation aus Ethanol/Methanol 3 : 1 gewonnen.

Farblose Kristalle vom Schmelzpunkt 250 - 252 °C

Rf : 0.59 ($CHCl_3$/ethanol. $NH_3$ [3.3 m] 8:2)

| | | | | |
|---|---|---|---|---|
| $C_{17}H_{23}N_9O_2$ (385) | Ber.: | C 52.98 | H 6.02 | N 32.71 |
| | Gef.: | C 53.07 | H 6.00 | N 32.61 |

$^1$H-NMR-Spektrum: ($d_6$-DMSO, TMS als interner Standard)

$\delta$ = 3.13 (m) ( 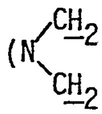 ) 4 H,

3.50 (s) (N-C$\underline{H}_3$) 3 H,

3.73 - 4.03 (m) ( 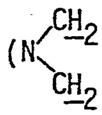 ) 4 H,

3.77 (s) (OC$\underline{H}_3$) 3 H,

3.81 (s) (OC$\underline{H}_3$) 3 H,

5.00 (s) (-N$\underline{H}_2$) 2 H (austauschbar mit $D_2O$)

6.77 (s) (Aromten-$\underline{H}$) 1 H,

7.13 (s, breit) (-N$\underline{H}_2$) 2 H (austauschbar mit $D_2O$)

7.47 (s) (Aromaten-$\underline{H}$) 1 H ppm.

Beispiel 6

4-Amino-2-[4-(4-amino-5-carbethoxythiazol-2-yl)-piperazin-1-yl]-6,7-dimethoxychinazolin.

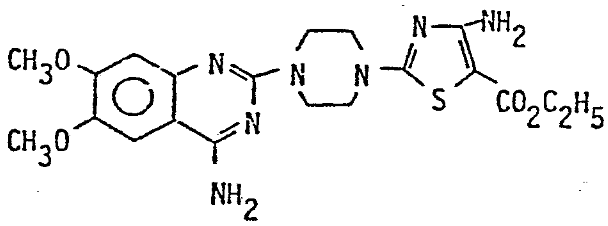

4.33 g (10 mmol) N-Cyano- (4-amino-6,7-dimethoxychinazolin-2-yl)-N,N-tetramethylen-4-imino -phenylisoharnstoff werden mit 1.6 ml (14.8 mmol) Thioglycolsäureethylester und 20 ml Triethylamin in 20 ml Methanol 7 Stunden unter Rückfluß erhitzt. Nach dem Abkühlen der Reaktionslösung auf Raumtemperatur wird das Reaktionsprodukt abgesaugt und aus Aceton umkristallisiert.

Farblose Kristalle vom Schmelzpunkt 189 - 190 °C

Ausbeute: 3.72 g (81 % d.Th.)

Rf: 0.52 ($CH_2Cl_2$ / $CH_3OH$ 90 : 10)

| | | | | |
|---|---|---|---|---|
| $C_{20}H_{25}N_7O_4S$ (450) | Ber.: | C 52.28 | H 5.48 | N 21.34 |
| | Gef.: | C 52.38 | H 5.47 | N 21.32 |

$^1$H-NMR-Spektrum: (d$_6$-DMSO, TMS als interner Standard)

$\delta$ = 1.20 (t) ($C\underline{H}_3$-$CH_2$) 3 H,
3.53 (m, breit) (-N($C\underline{H}_2$)$_2$) 4 H,
3.67 - 3.97 (m) (N(-$C\underline{H}_2$)$_2$) 4 H,
3.77 (s) ($OC\underline{H}_3$) 3 H,
3.83 (s) ($OC\underline{H}_3$) 3 H,
4.08 (q) ($CH_3C\underline{H}_2$) 2 H,
6.77 (s, breit) (Aromaten-$\underline{H}$, $N\underline{H}_2$) 3 H (austauschbar mit $D_2O$)
7.13 (s, breit) (-$N\underline{H}_2$) 2 H (austauschbar mit $D_2O$)
7.47 (s) (Aromaten-$\underline{H}$) 1 H ppm.

Beispiel 7

4-Amino-2-[4-(3-amino-1-methyl-1H-1,2,4-triazol-5-yl)homopiperazin-1-yl]-6,7-dimethoxy-chinazolin

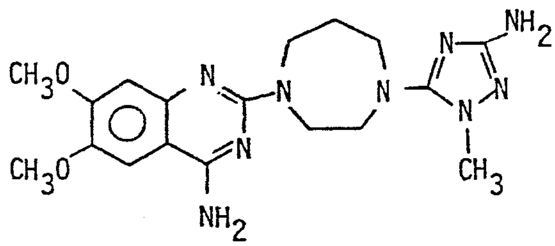

4.47 (10 mmol) N-Cyano-[(4-amino-6,7-dimethoxychinazolin-2-yl)-N,N-pentamethylen-4-imino]-phenylisoharnstoff werden mit 0,46 g (10 mmol) Methylhydrazin in 20 ml Methanol gelöst und 5 Stunden auf Rückflußtemperatur erhitzt, anschließend wird auf Raumtemperatur abgekühlt und die Reaktionslösung im Vakuum eingeengt. Der Rückstand wird säulenchromatographisch auf Kieselgel (Elutionsmittel $CHCl_3$/Triethylamin in EtOH 3.3 molar ) gereinigt.

Hellgelbe Kristalle vom Schmelzpunkt 128 - 132 °C

Rf: 0.58 ($CHCl_3$ / Triethylamin in EtOH [3,3 m] 70 : 30 )

$C_{18}H_{25}N_9O_2$ (399)

$^1$H-NMR-Spektrum: (d$_6$-DMSO, TMS als interner Standard)

$\delta$ = 1.92 (m) ($-CH_2$) 2 H,

3.13 - 3.50 (m) ($N<^{CH_2}_{CH_2}$) 4 H,

3.38 (s) ($-CH_3$) 3 H,

3.63 - 4.23 (m) ($N<^{CH_2}_{CH_2}$) 4 H,

3.77 (s) ($-OCH_3$) 3 H,

3.83 (s) ($-OCH_3$) 3 H,

4.87 (s) ($NH_2$) 2 H (austauschbar mit $D_2O$)

6.73 (s) (Aromaten-H) 1 H,
7.03 (s) ($NH_2$) 2 H (austauschbar mit $D_2O$),
7.43 (s) (Aromaten-H) 1 H ppm.

## Beispiel 8

4-Amino-2-[4-(5-amino-1-methyl-1H-1,2,4-triazol-3-yl)homopiperazin-1-yl]-6,7-dimethoxy-chinazolin

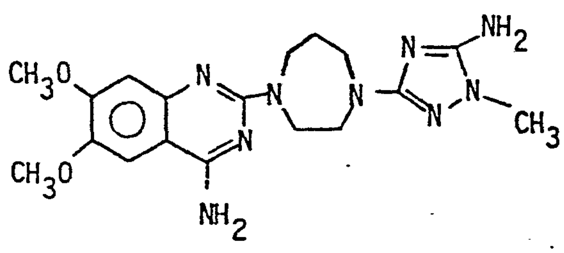

Die Herstellung erfolgt analog Beispiel 7. Die Isolierung gelingt durch säulenchromatographische Reinigung wie unter Beispiel 7 beschrieben.

Hellgelbe Kristalle vom Schmelzpunkt 208 ° (zers.)

Rf: 0.37 ($CHCl_3$ / Triethylamin in EtOH [3,3 m] 70 : 30 )

$C_{18}H_{25}N_9O_2$ (399)

$^1$H-NMR-Spektrum: (d$_6$-DMSO, TMS als interner Standard)

$\delta$ = 1.90 (m) ($-CH_2$) 2 H,
3.17 - 3.93 (2 x N<$CH_2$–$CH_2$) 8 H,
3.30 (s) ($-CH_3$) 3 H,
3.80 (s) ($-OCH_3$) 3 H,
5.83 (s) ($NH_2$) 2 H (austauschbar mit $D_2O$),

LUDWIG HEUMANN & CO. GMBH
8500 Nürnberg 1

Chinazolinderivate, Verfahren zu ihrer Herstellung und diese Verbindungen enthaltende Arzneimittel

PATENTANSPRÜCHE

1. Chinazolinderivate der allgemeinen Formel I:

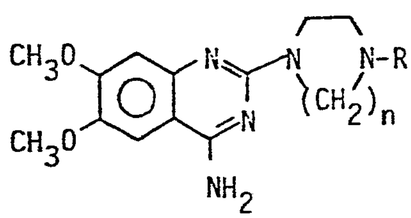

(I)

in der

R für einen ein- bis dreifach substituierten Fünfringheterocyclus aus der Gruppe Triazole, Oxadiazole, Thiazole und Imidazole steht, wobei der Heterocyclus mit dem Piperazin- bzw. Homopiperazinring über ein Kohlenstoffatom verbunden ist und n den Wert 2 oder 3 hat, oder

R eine N-Cyanophenylimidocarbonatgruppe bedeutet,

sowie die physiologisch annehmbaren Salze davon.

2. Verbindungen nach Anspruch 1, dadurch g e k e n n - z e i c h n e t , daß R einen Triazolring bedeutet, der in Position 3 durch eine Aminogruppe substituiert ist, die gegebenenfalls einen oder zwei lineare oder verzweigtkettige Niedrigalkylreste tragen kann.

3. Verbindungen nach Anspruch 1 oder 2, dadurch g e - k e n n z e i c h n e t , daß R einen Triazolring bedeutet, der in Position 1 durch eine lineare oder verzweigte Niedrigalkylkette substituiert ist und der in Position 3 eine Aminogruppe besitzt, die gegebenenfalls einen oder zwei lineare oder verzweigtkettige Niedrigalkylreste tragen kann.

4. Verbindungen nach Anspruch 1, dadurch g e k e n n - z e i c h n e t , daß R einen in den Positionen 4 und 5 durch eine Amino- und Carboxyniedrigalkylgruppe substituierten Thiazolring bedeutet.

5. Verbindungen nach Anspruch 1 bis 3, dadurch g e k e n n - z e i c h n e t , daß R einen Triazolring bedeutet, der in Position 1 durch eine lineare oder verzweigtkettige Niedrigalkylkette substituiert ist und der in Position 5 eine Aminogruppe besitzt, die gegebenenfalls einen oder zwei lineare oder verzweigtkettige Niedrigalkylreste tragen kann.

6. Verbindungen nach Anspruch 1, dadurch g e k e n n - z e i c h n e t , daß R einen in Position 4 durch eine niedrigalkoxysubstituierten 1,2,5-Thiadiazol-1-oxidring bedeutet.

7. Verbindungen nach Anspruch 1, dadurch g e k e n n - z e i c h n e t , daß R für eine N-Cyanophenylimidocarbonatgruppe steht.

8. N-Cyano-[(4-amino-6,7-dimethoxychinazolin-2-yl)-N,N-tetramethylen-4-imino]-phenylisoharnstoff und dessen physiologisch annehmbaren Salze.

9. 4-Amino-2-[4-(3-amino-1-methyl-1H-1,2,4-triazol-5-yl)-piperazin-1-yl]-6,7-dimethoxychinazolin und dessen physiologisch annehmbaren Salze.

10. 4-Amino-2- 4-(5-amino-1-methyl-1H-1,2,4-triazol-3-yl piperazin-1-yl -6,7-dimethoxy-chinazolin und dessen physiologisch annehmbaren Salze.

11. Verfahren zur Herstellung der Chinazolinderivate nach den Ansprüchen 1 bis 8, dadurch g e k e n n z e i c h n e t , daß man

a) zur Herstellung von Verbindungen der allgemeinen Formel I, in der R für

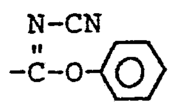

steht, eine Verbindung der Formel II

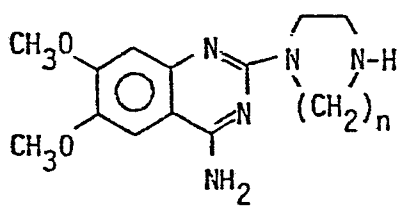

(II)

mit N-Cyanodiphenylimidocarbonat der Formel III

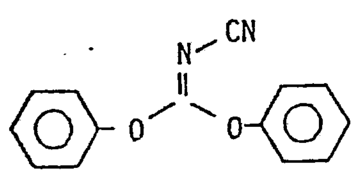

(III)

zu einer erfindungsgemäßen Verbindung der allgemeinen Formel Ia

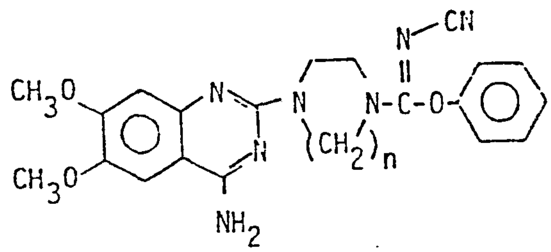

(Ia)

umsetzt

oder daß man

b) zur Herstellung von Verbindungen der allgemeinen Formel I, in der R für ein 1,2,4-Triazol oder 1,3-Thiazol steht, eine Verbindung der allgemeinen Formel Ia in einem alkoholischen Lösungsmittel mit einem Hydrazinderivat oder Mercaptoessigsäureester zu den erfindungsgemäßen Verbindungen der allgemeinen Formel I cyclisiert

und daß man gegebenenfalls die in Stufe a) oder b) erhaltene Verbindung in ihr physiologisch annehmbares Salz umwandelt.

12. Arzneimittel, dadurch gekennzeichnet, daß es eine Verbindung nach den Ansprüchen 1 bis 8 zusammen mit einem inerten pharmazeutisch annehmbaren Träger oder einem inerten, pharmazeutisch annehmbaren Verdünnungsmittel enthält.